# EUROPEAN PATENT APPLICATION

(11) **EP 2 169 059 A1**
(43) Date of publication of application: **31.03.2010**
(21) Application number: 08165168.9
(22) Date of filing: 25.09.2008
(51) Int. Cl.: C12N 15/11, A61K 31/713, C12Q 1/68, A61P 9/00, A61P 17/00

(54) **Means and methods for counteracting, preventing and/or determining fibrosis or a risk of fibrosis**

(71) Applicant: Academisch Medisch Centrum bij de Universiteit van Amsterdam, 1105 AZ Amsterdam (NL); Academisch Ziekenhuis Maastricht, 6229 HX Maastricht (NL); Universiteit Maastricht, 6211 LK Maastricht (NL)
(72) Inventor: Creemers, Esther Elisa Johanna Maria, 1058 TS Amsterdam (NL); Pinto, Yigal-Martin, 1186 BR Amstelveen (NL); Duisters, Rudy Franciscus Johannes Josephus, 6021 ZS Budel (NL)
(74) Representative: Hatzmann, Martin

(57) **Abstract**

The present invention provides means and methods for counteracting, treating, diminishing, delaying, preventing and/or determining (a risk of) fibrosis.

## Description

The invention relates to the fields of biology and medicine.

Fibrosis is the formation or development of excess fibrous connective tissue in an organ or tissue as a reparative or reactive process. It involves excessive production of extracellular matrix (ECM) proteins, such as collagen. A well-organized extracellular matrix (ECM) is necessary to maintain strength and functional integrity of tissue and is involved in communication between different cells. Fibrosis is often caused by injury or disease. In response to numerous pathological stimuli ECM proteins accumulate excessively. This process alters mechanical stiffness, which adversely affects the affected tissue or organ. Fibrosis occurs in various different tissues and organs. Non-limiting examples include renal fibrosis, liver fibrosis, pulmonary fibrosis and cardiac fibrosis. If left untreated, fibrosis often ultimately leads to loss of function.

Connective tissue growth factor (CTGF) is a secreted protein which has been identified as a powerful inducer of ECM synthesis. The importance of CTGF is recognized in many different forms of pathology and has been described in different organs, including the heart, to be an important mediator of tissue fibrosis. CTGF has thus emerged as a new target for the therapeutic intervention in fibrotic diseases. CTGF expression is induced by TGF6 and other pro-hypertrophic stimuli such as endothelin. Regulation of CTGF expression at the promoter level has been studied extensively. TGF-6 is one of the most potent inducers of CTGF and regulates expression through a signaling cascade requiring Smads, PKC ras/MEK/ERK and an Ets-1 binding element in the CTGF promoter. However, little is known as to how CTGF transcripts are regulated at the posttranscriptional level.

It is an object of the present invention to provide means and methods for counteracting, preventing or determining (a risk of) fibrosis. It is a further object to provide means and methods for influencing expression of connective tissue growth factor (CTGF) in a cell.

Accordingly, the present invention provides a method for influencing expression of CTGF in a cell, comprising:
- influencing the expression, amount and/or activity of miRNA-30 and/or miRNA-133, or a functional part or derivative thereof, within said cell, and/or
- influencing interaction of CTGF mRNA with miRNA-30 and/or miRNA-133, or a functional part or derivative thereof, within said cell.

The present invention provides the insight that miRNA-30 and miRNA-133 are powerful negative regulators of CTGF expression. MicroRNAs (miRNAs) are small RNA molecules encoded in the genomes of plants and animals. They are present within introns of protein-coding genes, in polycistronic transcripts encoding multiple miRNAs and in individual miRNA genes. These highly conserved RNAs with a length of approximately 21-23 nucleotides usually regulate the expression of genes by binding to the 3'-untranslated regions (3'-UTRs) of specific mRNAs. Each miRNA is thought to regulate multiple genes, and since hundreds of miRNA genes are predicted to be present in higher eukaryotes the potential regulatory circuitry afforded by miRNA is enormous. Several research groups have provided evidence that miRNAs act as key regulators of processes as diverse as early development, cell proliferation and cell death, apoptosis and fat metabolism, and cell differentiation. There is speculation that in higher eukaryotes, the role of miRNAs in regulating gene expression could be as important as that of transcription factors.

miRNAs are transcribed by RNA polymerase II. The primary transcripts, which generally have a length of several kilobases, are called pri-miRNAs. Pri-miRNAs are processed in the cell nucleus to shorter, 70-100 nucleotide stem-loop structures known as pre-miRNAs. This processing is performed in animals by the RNase III endonuclease Drosha. Pre-miRNAs are subsequently transported into the cytoplasm, where they are processed to double-stranded miRNAs with a length of 21-23 nucleotides by a second RNase III endonuclease, DICER. One strand of the miRNA duplex is subsequently incorporated into the RNA-induced silencing complex (RISC). As part of the RISC, gene expression of a target gene is counteracted by inhibiting translation and/or by cleaving mRNA.

According to the present invention, CTGF is a target gene of miRNA-30 and miRNA-133. miRNA-30 and miRNA-133 counteract expression of CTGF. Expression of CTGF in a cell is thus influenced by influencing the expression, amount and/or activity of miRNA-30 and/or miRNA-133, or a functional part or derivative thereof, within said cell, and/or by influencing interaction of CTGF mRNA with miRNA-30 and/or miRNA-133, or a functional part or derivative thereof, within said cell. A use of miRNA-30 and/or miRNA-133 or a functional part or derivative thereof, or use of a compound capable of influencing the expression, amount and/or activity of miRNA-30 and/or miRNA-133 in a cell, or use of a compound capable of influencing interaction of CTGF mRNA with miRNA-30 and/or miRNA-133, for influencing expression of CTGF in said cell is therefore also provided. In one embodiment, expression of CTGF is influenced *in vitro,* for instance in a cell culture.

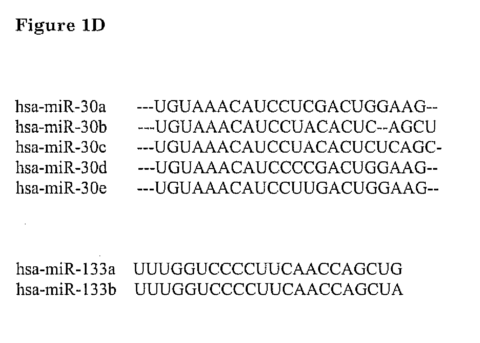

Sequences of human miRNA-30, as well as sequences of human miRNA-133, are depicted in Figure 1D. These sequences are conserved in (at least) mice and humans. As currently known, miRNA-133 has two isoforms (miRNA-133a and miRNA-133b). According to the present invention, both miRNA-133a and miRNA-133b are capable of counteracting expression of CTGF. Hence, as used herein, the term "miRNA-133" encompasses any isoform of miRNA-133. The miRNA30 family also has several members (currently known are miRNA-30a, miRNA-30b, miRNA-30c, miRNA-30d and miRNA-30e). According to the present invention, all members of the miRNA-30 family are capable of counteracting expression of CTGF. Hence, as used herein, the term "miRNA-30" encompasses any miRNA-30 member. Said term preferably at least encompasses miRNA-30c, since this member is very well capable of counteracting CTGF expression. Although miRNA-30 and miRNA-133 are known in the art, their effect upon CTGF expression was unknown before the present invention.

Now that the invention has provided the insight that miRNA-30 and miRNA-133 are capable of counteracting expression of CTGF, it has become possible to counteract or prevent CTGF-related diseases. A CTGF-related disease is defined herein as a disease which is at least in part, directly or indirectly, caused by the presence and/or action of CTGF. Counteracting the expression of CTGF will result in a lower amount of CTGF, which in turn will diminish the onset, progression and/or effects of a CTGF-related disease. One important condition which is mediated by CTGF is fibrosis. CTGF is an important mediator of tissue fibrosis because it induces ECM synthesis. According to the present invention, miRNA-30 and/or miRNA-133 are capable of counteracting fibrosis by counteracting CTGF expression.

One embodiment therefore provides a method for counteracting, treating, diminishing, delaying and/or preventing fibrosis in a subject, the method comprising:
- increasing the expression, amount and/or activity of miRNA-30 and/or miRNA-133, or a functional part or derivative thereof, within said subject, and/or
- increasing interaction of CTGF mRNA with miRNA-30 and/or miRNA-133, or with a functional part or derivative thereof, within said subject. As described herein before, said miRNA-30 and/or miRNA-133 comprises any member of the miRNA-30 and/or miRNA-133 family. Preferably, said miRNA-30 at least comprises miRNA-30c and said miRNA-133 preferably at least comprises miRNA-133a and/or miRNA-133b. These members are very well capable of counteracting CTGF expression.

In one embodiment, said subject has been diagnosed with fibrosis. In another embodiment, however, said subject has an increased risk of fibrosis, for instance because said subject is already suffering from injury or disease. In an alternative embodiment, a method according to the invention is performed in order to generally prevent fibrosis in an unaffected subject.

Methods for diagnosing fibrosis are known in the art. A subject is for instance diagnosed with fibrosis by determining whether mechanical stiffness is increased, whether normal functions, such as for instance electrical properties of the heart, are decreased, whether ECM proteins are accumulated excessively and/or whether CTGF levels are above a certain threshold level. An increased risk of fibrosis is often present in a subject when the subject is suffering from an injury or disorder.

Methods of the invention are suitable for use against any CTGF-related disease. Said disease for instance comprises pulmonary fibrosis, liver fibrosis and/or renal fibrosis. In one preferred embodiment a method according to the invention is used in order to counteract, treat, diminish, delay and/or prevent cardiac fibrosis. Cardiac fibrosis is a serious condition which alters mechanical and electrical properties of the heart. This results in impaired heart functioning. miRNA-30 and miRNA-133 are significantly expressed in cardiac tissue and thus already play a regulatory role *in vivo.* While miRNA-133 is specific for cardiac myocytes, miRNA-30, which is expressed in the heart at equivalent levels to that of miR-133, is also expressed in cardiac fibroblasts. Further provided is therefore a method for counteracting, treating, diminishing, delaying and/or preventing cardiac fibrosis in a subject, the method comprising:
- increasing the expression, amount and/or activity of miRNA-30 and/or miRNA-133, or a functional part or derivative thereof, within said subject, and/or
- increasing interaction of CTGF mRNA with miRNA-30 and/or miRNA-133, or with a functional part or derivative thereof, within said subject.

In one preferred embodiment, said subject has been diagnosed with cardiac fibrosis or with a risk of cardiac fibrosis. In an alternative embodiment, said method is performed in order to generally prevent cardiac fibrosis in an unaffected subject.

It is possible to increase the expression, amount and/or activity of miRNA-30 and/or miRNA-133 directly, for instance by administering an activating compound capable of increasing the expression and/or activity of miRNA-30 and/or miRNA-133. It is also possible to increase the expression, amount and/or activity of miRNA-30 and/or miRNA-133 indirectly, for instance by decreasing the expression, amount and/or activity of an inhibitor of miRNA-30 or miRNA-133.

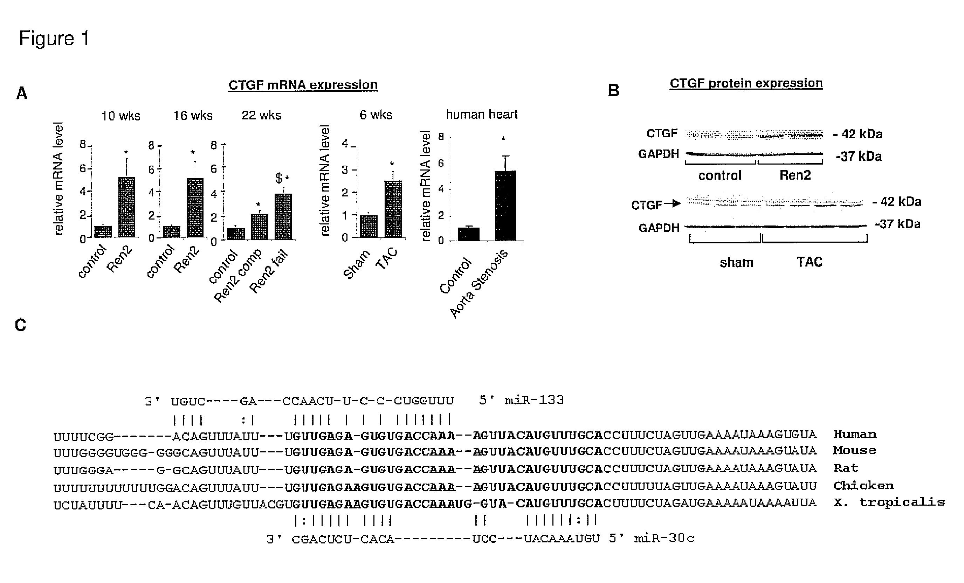

In a preferred embodiment, the amount of miRNA-30 and/or miRNA-133 is increased by administering pri-miRNA-30 and/or pri-miRNA-133 and/or pre-miRNA-30 and/or pre-miRNA-133 and/or miRNA-30 and/or miRNA-133, or a functional part or derivative thereof, to said subject. Administration of any of these compounds, or any combination of these compounds, results in (increased) counteraction of CTGF and, thus, counteraction and/or prevention of fibrosis. Pri-miRNA-30 and pri-miRNA-133 are the primary transcripts, having a length of several kilobases, which are obtained after transcription of the genomic miRNA-30 and miRNA-133 sequences, respectively, by RNA polymerase II. Pre-miRNA-30 and pre-miRNA-133 are the shorter, 70-100 nucleotide stem-loop structures which are obtained after processing of pri-miRNA-30 and pri-miRNA-133, respectively, by Drosha. The sequences of miRNA-30 and miRNA-133 are depicted in Figure 1.

A functional part of pri-miRNA-30 or pri-miRNA-133 or pre-miRNA-30 or pre-miRNA-133 or miRNA-30 or miRNA-133 is defined herein as a nucleic acid sequence with a length of at least 7 nucleotides which is shorter then pri-miRNA-30 or pri-miRNA-133 or pre-miRNA-30 or pre-miRNA-133 or miRNA-30 or miRNA-133, respectively, and which is capable of binding a CTGF gene and/or CTGF mRNA and counteracting CTGF expression. Said functional part is preferably capable of binding the same 3' UTR region of CTGF which is capable of being bound by miRNA-30 or miRNA-133. Said functional part preferably comprises a sequence of the seed region of miRNA-30 or miRNA-133. A seed region of miRNA-30 or miRNA-133 is present in the 5' region of naturally occurring miRNA-30 or miRNA-133, respectively. Said seed region is particularly involved in target recognition. The seed region of miRNA-30 members a, b, c, d and e has the same sequence. It comprises the sequence GUAAACA. The seed region of miRNA-133 members a and b is also the same and comprises the sequence UUGGUCC. A functional part of pri-miRNA-30 or pri-miRNA-133 or pre-miRNA-30 or pre-miRNA-133 or miRNA-30 or miRNA-133 is thus preferably a nucleic acid sequence with a length of at least 7 nucleotides, comprising the sequence GUAAACA and/or UUGGUCC. However, slight modifications of said seed region are allowed, as long as the capability of binding a CTGF gene or CTGF mRNA is maintained. A functional part of pri-miRNA-30 or pri-miRNA-133 or pre-miRNA-30 or pre-miRNA-133 or miRNA-30 or miRNA-133 thus also comprises a nucleic acid sequence with a length of at least 7 nucleotides, comprising a sequence which has at least 72%, more preferably at least 80%, more preferably at least 86%, most preferably at least 90% sequence identity with the sequence GUAAACA and/or UUGGUCC.

A functional derivative of pri-miRNA-30 or pri-miRNA-133 or pre-miRNA-30 or pre-miRNA-133 or miRNA-30 or miRNA-133 is defined herein as a molecule which comprises a sequence which has 70% or more, but less than 100%, sequence identity with pri-miRNA-30 or pri-miRNA-133 or pre-miRNA-30 or pre-miRNA-133 or miRNA-30 or miRNA-133 and which has at least one same property in common irrespective of quantitative considerations. Said functional equivalent is capable of binding a CTGF gene and/or CTGF mRNA and counteracting CTGF expression, albeit not necessarily to the same extent as pri-miRNA-30 or pri-miRNA-133 or pre-miRNA-30 or pre-miRNA-133 or miRNA-30 or miRNA-133. Such functional equivalent for instance comprises:
- a polynucleotide wherein one or more amino acid residues are added to the native sequence of pri-miRNA-30 or pri-miRNA-133 or pre-miRNA-30 or pre-miRNA-133 or miRNA-30 or miRNA-133;
- a pri-miRNA-30 or pri-miRNA-133 or pre-miRNA-30 or pre-miRNA-133 or miRNA-30 or miRNA-133 molecule wherein between 1-40 % of the nucleotides are substituted by one or more other nucleotides, as long as the seed region sequence retains at least 72% sequence identity with the sequence GUAAACA or UUGGUCC; and/or
- a pri-miRNA-30 or pri-miRNA-133 or pre-miRNA-30 or pre-miRNA-133 or miRNA-30 or miRNA-133 derivative wherein at least one nucleotide has been modified so that the resulting product has a non-naturally occurring nucleotide. Preferably, a functional equivalent of pri-miRNA-30 or pri-miRNA-133 or pre-miRNA-30 or pre-miRNA-133 or miRNA-30 or miRNA-133 has a nucleotide sequence having at least about 75% sequence identity with pri-miRNA-30 or pri-miRNA-133 or pre-miRNA-30 or pre-miRNA-133 or miRNA-30 or miRNA-133, preferably at least about 80%, more preferably at least about 85%, more preferably at least about 90%, most preferably at least about 95%. The higher the sequence identity, the more closely said functional equivalent resembles pri-miRNA-30 or pri-miRNA-133 or pre-miRNA-30 or pre-miRNA-133 or miRNA-30 or miRNA-133.

A preferred example of a functional equivalent of pri-miRNA-30 or pri-miRNA-133 or pre-miRNA-30 or pre-miRNA-133 or miRNA-30 or miRNA-133 is a vector comprising at least the seed region of miRNA-30 or miRNA-133, or a vector comprising a sequence which has at least 72%, preferably at least 80%, more preferably at least 86%, most preferably at least 90% sequence identity with the sequence GUAAACA and/or UUGGUCC.

The term "% sequence identity" is defined herein as the percentage of nucleotides in a nucleic acid sequence that is identical with the nucleotides in a nucleic aid sequence of interest, after aligning the sequences and optionally introducing gaps, if necessary, to achieve the maximum percent sequence identity. Methods and computer programs for alignments are well known in the art. As used herein, the terms "nucleic acid sequence" and "nucleotides" also encompass non-natural molecules based on and/or derived from nucleic acid sequences, such as for instance artificially modified nucleic acid sequences, peptide nucleic acids, as well as nucleic acid sequences comprising at least one modified nucleotide and/or non-natural nucleotide such as for instance inosine.

Methods for introducing polynucleotides into a cell are known in the art. Methods for introducing nucleic acid for instance comprise calcium phosphate transfection, DEAE-Dextran, electroporation or liposome-mediated transfection. Alternatively, direct injection of the polynucleotide is employed. Preferably however, a nucleic acid sequence is introduced into a cell by a vector, preferably a viral vector. Said vector preferably comprises a retroviral, adenoviral, adeno-associated viral (AAV), or lentiviral vector. In one embodiment an AAV9 vector is used.

Various terms are known in the art which refer to introduction of nucleic acid into a cell by a vector. Examples of such terms are "transduction", "transfection" and "transformation". Techniques for generating a vector with a nucleic acid sequence and for introducing said vector into a cell are known in the art.

Preferably, a pri-miRNA-30 or pri-miRNA-133 or pre-miRNA-30 or pre-miRNA-133 or miRNA-30 or miRNA-133 or a functional part or derivative thereof is used which is able to be introduced into a mammalian cell *in vivo.* Non-limiting examples of methods according to the invention are the coupling of said nucleic acid sequence to cell-penetrating peptides, to microcarriers or to nanocarriers, or the use of liposomes containing said nucleic acid sequence. Preferably, said inhibitor is targeted to heart muscle cells, for instance by using artificial HDL-like particles bound to said inhibitor, enhancing delivery to the myocardium.

According to the present invention, fibrosis is particularly well counteracted and/or prevented by administering pri-miRNA-30 and/or pri-miRNA-133 and/or pre-miRNA-30 and/or pre-miRNA-133 and/or miRNA-30 and/or miRNA-133, or a functional part or derivative thereof, to a subject suffering from, or at risk of suffering from, fibrosis. Fibrosis is also counteracted and/or prevented by administering a compound capable of increasing or restoring the expression, amount and/or activity of miRNA-30 and/or miRNA-133 in said subject, or by administering a compound capable of increasing interaction of CTGF mRNA with miRNA-30 and/or miRNA-133. The invention therefore further provides pri-miRNA-30 and/or pri-miRNA-133 and/or pre-miRNA-30 and/or pre-miRNA-133 and/or miRNA-30 and/or miRNA-133, or a functional part or derivative thereof, or a compound capable of increasing or restoring the expression, amount and/or activity of miRNA-30 and/or miRNA-133 in a cell, or a compound capable of increasing interaction of CTGF mRNA with miRNA-30 and/or miRNA-133, for use in counteracting, treating, diminishing, delaying and/or preventing fibrosis. Said fibrosis preferably comprises pulmonary fibrosis, liver fibrosis and/or renal fibrosis. Most preferably, said fibrosis comprises cardiac fibrosis.

The above mentioned compounds, or any combination thereof, are thus particularly suitable for the preparation of a medicament or prophylactic agent against fibrosis. Further provided is therefore a use of pri-miRNA-30 and/or pri-miRNA-133 and/or pre-miRNA-30 and/or pre-miRNA-133 and/or miRNA-30 and/or miRNA-133, or a functional part or derivative thereof, or a use of a compound capable of increasing or restoring the expression, amount and/or activity of miRNA-30 and/or miRNA-133 in a cell, or a use of a compound capable of increasing interaction of CTGF mRNA with miRNA-30 and/or miRNA-133, for the preparation of a medicament or prophylactic agent against fibrosis. As described before, said miRNA-30 preferably at least comprises miRNA-30c and said miRNA-133 preferably at least comprises miRNA-133a and/or miRNA-133b, since these members are particularly well capable of counteracting CTGF expression. Said fibrosis preferably comprises pulmonary fibrosis, liver fibrosis and/or renal fibrosis. Most preferably, said fibrosis comprises cardiac fibrosis. Said prophylactic agent is particularly suitable for subjects with an increased risk of fibrosis, for instance subjects already suffering from injury or disease. However, said prophylactic agent is also suitable for generally preventing fibrosis in unaffected subjects.

Administration of any of the above mentioned compounds, or any combination thereof, to a subject is particularly suitable for counteracting and/or preventing fibrosis in said subject. Further provided is therefore a method for counteracting, treating, diminishing, delaying and/or preventing fibrosis, preferably pulmonary fibrosis, liver fibrosis and/or renal fibrosis, most preferably cardiac fibrosis, in a subject, the method comprising:
- administering to the subject a therapeutically effective amount of pri-miRNA-30 and/or pri-miRNA-133 and/or pre-miRNA-30 and/or pre-miRNA-133 and/or miRNA-30 and/or miRNA-133, or a functional part or derivative thereof, and/or
- administering to the subject a therapeutically effective amount of a compound capable of increasing or restoring the expression, amount and/or activity of miRNA-30 and/or miRNA-133 in said subject, and/or
- administering to the subject a therapeutically effective amount of a compound capable of increasing interaction of CTGF mRNA with miRNA-30 and/or miRNA-133.

In one embodiment, any of the above mentioned compounds, or any combination thereof, is administered to a subject who has been diagnosed with fibrosis. In another embodiment, any of the above mentioned compounds, or any combination thereof, is administered to a subject with an increased risk of fibrosis, for instance a subject who is already suffering from injury or disease. However, said compounds, or any combination thereof, are also suitable for generally preventing fibrosis in unaffected subjects.

As already described, methods for establishing the presence or risk of fibrosis include examination of mechanical stiffness, organ functioning, accumulation of ECM proteins and CTGF levels. However, now that the present invention has disclosed the presence of a connection between miRNA-30/miRNA-133 and fibrosis, additional methods have become available. According to the invention, the presence or risk of fibrosis is established by determining whether the amount of pri-miRNA-30 and/or pri-miRNA-133 and/or pre-miRNA-30 and/or pre-miRNA-133 and/or miRNA-30 and/or miRNA-133 in a sample of a subject is below a certain reference value. In one embodiment said reference value represents the amount of pri-miRNA-30 and/or pri-miRNA-133 and/or pre-miRNA-30 and/or pre-miRNA-133 and/or miRNA-30 and/or miRNA-133 in a healthy subject or healthy population. If the amount of pri-miRNA-30 and/or pri-miRNA-133 and/or pre-miRNA-30 and/or pre-miRNA-133 and/or miRNA-30 and/or miRNA-133 in a sample of a subject is significantly below the reference value, CTGF expression is not sufficiently counteracted. As a result, CTGF expression will be too high and the subject is suffering from, or at risk of suffering from, fibrosis. In such case, a therapy according to the present invention is recommended.

On the other hand, if the amount of pri-miRNA-30 and/or pri-miRNA-133 and/or pre-miRNA-30 and/or pre-miRNA-133 and/or miRNA-30 and/or miRNA-133 in a sample of a subject is essentially the same as, or higher than, the reference value, CTGF expression is sufficiently counteracted. In this case, the subject is not diagnosed as suffering from, or at risk of suffering from, fibrosis.

Of course, other kinds of reference values can be used. For instance, a reference value can be used that represents the amount of pri-miRNA-30 and/or pri-miRNA-133 and/or pre-miRNA-30 and/or pre-miRNA-133 and/or miRNA-30 and/or miRNA-133 in a subject or population suffering from fibrosis. In this case, a sample with an amount of pri-miRNA-30 and/or pri-miRNA-133 and/or pre-miRNA-30 and/or pre-miRNA-133 and/or miRNA-30 and/or miRNA-133 which is essentially the same as said reference value indicates (a risk of) fibrosis. A sample wherein said value is much smaller indicates health.

The invention therefore provides a method for determining whether a subject is suffering from fibrosis, or whether a subject has an increased risk of developing fibrosis, the method comprising:
- obtaining a sample from said subject;
- determining the amount of pri-miRNA-30 and/or pri-miRNA-133 and/or pre-miRNA-30 and/or pre-miRNA-133 and/or miRNA-30 and/or miRNA-133 in said sample;
- comparing said amount with a reference; and
- determining from said comparison whether or not the subject is suffering from fibrosis or is having an increased risk of developing fibrosis.

Said fibrosis preferably comprises cardiac fibrosis, since miRNA-30 and miRNA-133 are significantly expressed in cardiac tissue.

The amount of pri-miRNA-30 and/or pri-miRNA-133 and/or pre-miRNA-30 and/or pre-miRNA-133 and/or miRNA-30 and/or miRNA-133 in a sample of a subject is preferably established using a binding compound. At least part of a sample is preferably contacted with such binding compound (optionally after previous processing of the sample), where after unbound components are preferably washed away and bound compounds are preferably visualized and quantified. One embodiment thus provides a method according to the invention for determining whether a subject is suffering from fibrosis, or whether a subject has an increased risk of developing fibrosis, the method further comprising contacting at least part of said sample with at least one compound capable of specifically binding pri-miRNA-30 and/or pri-miRNA-133 and/or pre-miRNA-30 and/or pre-miRNA-133 and/or miRNA-30 and/or miRNA-133. Of course, if only a part of said sample is used, a part is used which contains microRNA so that a suitable test is carried out.

The invention furthermore provides a kit of parts for carrying out a method according to the present invention. Further provided is thus a kit of parts comprising:
- at least one compound capable of specifically binding pri-miRNA-30 and/or pri-miRNA-133 and/or pre-miRNA-30 and/or pre-miRNA-133 and/or miRNA-30 and/or miRNA-133; and
- instructions for use of said compound for determining whether a subject is suffering from fibrosis, preferably pulmonary fibrosis, liver fibrosis and/or renal fibrosis, more preferably cardiac fibrosis, or whether a subject has an increased risk of developing fibrosis, preferably pulmonary fibrosis, liver fibrosis and/or renal fibrosis, more preferably cardiac fibrosis.

The invention furthermore provides a kit of parts for carrying out a therapy according to the present invention. With such kit of part, CTGF expression is typically counteracted via miRNA-30 and/or miRNA-133. A kit of parts according to the invention comprises:
- pri-miRNA-30 and/or pri-miRNA-133 and/or pre-miRNA-30 and/or pre-miRNA-133 and/or miRNA-30 and/or miRNA-133, or a functional part or derivative thereof, and/or
- a compound capable of increasing or restoring the expression, amount and/or activity of miRNA-30 and/or miRNA-133 in a cell, and/or
- a compound capable of increasing interaction of CTGF mRNA with miRNA-30 and/or miRNA-133, and
- instructions for use of said compound(s) for counteracting, treating, diminishing, delaying and/or preventing fibrosis, preferably pulmonary fibrosis, liver fibrosis and/or renal fibrosis, more preferably cardiac fibrosis, in a subject in need thereof. Said pri-miRNA-30 and/or pri-miRNA-133 and/or pre-miRNA-30 and/or pre-miRNA-133 and/or miRNA-30 and/or miRNA-133, or a functional part or derivative thereof is preferably present in a vector. It is also possible to use a vector comprising a nucleic acid sequence encoding any of the above mentioned compounds. Said vector preferably comprises a viral vector, most preferably a retroviral, adenoviral, adeno-associated viral or lentiviral vector. In one embodiment an AAV9 vector is used.

Further provided is therefore a vector comprising:
- pri-miRNA-30 and/or pri-miRNA-133 and/or pre-miRNA-30 and/or pre-miRNA-133 and/or miRNA-30 and/or miRNA-133, or a functional part or derivative thereof, and/or
- a nucleic acid sequence encoding a compound capable of increasing or restoring the expression, amount and/or activity of miRNA-30 and/or miRNA-133 in a cell, and/or
- a nucleic acid sequence encoding a compound capable of increasing interaction of CTGF mRNA with miRNA-30 and/or miRNA-133.

In one preferred embodiment, said vector comprises:
- pri-miRNA-30 and/or pre-miRNA-30 and/or miRNA-30, or a functional part or derivative thereof, and/or
- a nucleic acid sequence encoding a compound capable of increasing or restoring the expression, amount and/or activity of miRNA-30 in a cell, and/or
- a nucleic acid sequence encoding a compound capable of increasing interaction of CTGF mRNA with miRNA-30. Such vector is particularly suitable for counteracting cardiac fibrosis, since miRNA-30 is expressed in cardiac myocytes as well as in cardiac fibroblasts.

A vector according to the invention preferably comprises a retroviral, adenoviral, adeno-associated viral or lentiviral vector. In one embodiment, said vector comprises an AAV9 vector.

Any of the above mentioned nucleic acid sequences of a vector according to the invention are preferably operably linked to a promoter, so that expression of said nucleic acid will occur after transfection. Said promoter is preferably suitable for expression in a mammalian cell. In a particularly preferred embodiment, a vector according to the invention is suitable for expression in a cardiac cell, so that (a risk of) cardiac fibrosis is counteracted. In that case said vector preferably comprises a promoter suitable for expression in a cardiac cell. In another embodiment, however, a vector according to the invention comprises a ubiquitous promoter. It is advantageous to use an inducible promoter, so that expression of (at least one) nucleic acid of a vector according to the invention is regulated at will. Non-limiting examples of inducible promoters are cardiac troponin and alpha myosin heavy chain, which are cardiomyocyte specific promoters. In one embodiment a cardiac fibroblast specific promoter is used.

A vector according to the invention is particularly suitable for therapy. Further provided is therefore a use of a vector comprising:
- pri-miRNA-30 and/or pri-miRNA-133 and/or pre-miRNA-30 and/or pre-miRNA-133 and/or miRNA-30 and/or miRNA-133, or a functional part or derivative thereof, and/or
- a nucleic acid sequence encoding a compound capable of increasing or restoring expression, amount and/or activity of miRNA-30 and/or miRNA-133 in a cell, and/or
- a nucleic acid sequence encoding a compound capable of increasing interaction of CTGF mRNA with miRNA-30 and/or miRNA-133,
for the preparation of a medicament and/or prophylactic agent for counteracting, treating, diminishing, delaying and/or preventing fibrosis, preferably pulmonary fibrosis, liver fibrosis and/or renal fibrosis, more preferably cardiac fibrosis.

Also provided is a method for counteracting, treating, diminishing, delaying and/or preventing fibrosis, preferably pulmonary fibrosis, liver fibrosis and/or renal fibrosis, more preferably cardiac fibrosis, in a subject, the method comprising administering a therapeutically amount of a vector according to the invention to the subject. As said before, said vector preferably comprises a retroviral, adenoviral, adeno-associated viral or lentiviral vector. In one embodiment, an AAV9 vector is used. An isolated or recombinant cell comprising a vector according to the invention is also herewith provided.

It is possible to provide cells with pri-miRNA-30 and/or pri-miRNA-133 and/or pre-miRNA-30 and/or pre-miRNA-133 and/or miRNA-30 and/or miRNA-133 *in vitro.* Such cells are particularly suitable for research purposes. Moreover, such cells are suitable for therapy. For instance, cells overexpressing miRNA-30 and/or miRNA-133 are administered to a subject in order to form non-fibrotic tissue *in vivo.* Further provided is therefore a use of an isolated cell capable of overexpressing miRNA-30 and/or miRNA-133, or a functional part or derivative thereof, for the preparation of a medicament and/or prophylactic agent for counteracting, treating, diminishing, delaying and/or preventing fibrosis, preferably pulmonary fibrosis, liver fibrosis and/or renal fibrosis, more preferably cardiac fibrosis.

Pharmaceutical compositions allowing counteraction of CTGF expression by miRNA-30 and/or miRNA-133 are also provided herein.

A pharmaceutical composition according to the invention comprises:
- pri-miRNA-30 and/or pri-miRNA-133 and/or pre-miRNA-30 and/or pre-miRNA-133 and/or miRNA-30 and/or miRNA-133, or a functional part or derivative thereof, and/or
- a compound capable of increasing or restoring the expression, amount and/or activity of miRNA-30 and/or miRNA-133 in a cell, and/or
- a compound capable of increasing interaction of CTGF mRNA with miRNA-30 and/or miRNA-133, and/or
- a vector according to the invention, and/or
- a cell capable of overexpressing miRNA-30 and/or miRNA-133, or a functional part or derivative thereof,
together with a pharmaceutically acceptable carrier, diluent or excipient.

Also provided is a method for counteracting, treating, diminishing, delaying and/or preventing fibrosis, preferably pulmonary fibrosis, liver fibrosis and/or renal fibrosis, more preferably cardiac fibrosis, in a subject, the method comprising administering a therapeutically amount of a pharmaceutical composition according to the invention to the subject.

Further provided is a non-human animal comprising:
- an exogenous nucleic acid sequence comprising pri-miRNA-30 and/or pri-miRNA-133 and/or pre-miRNA-30 and/or pre-miRNA-133 and/or miRNA-30 and/or miRNA-133, or a functional part or derivative thereof, and/or
- an exogenous nucleic acid sequence encoding a compound capable of increasing or restoring the expression, amount and/or activity of miRNA-30 and/or miRNA-133 in a cell, and/or
- an exogenous nucleic acid sequence encoding a compound capable of increasing interaction of CTGF mRNA with miRNA-30 and/or miRNA-133.

Said non-human animal preferably comprises a mammal. In one preferred embodiment said non-human animal comprises a test animal such as a rodent, a rabbit, a goat, a cow, a sheep or a monkey. A non-human animal which has been provided with a vector, an isolated cell, and/or a pharmaceutical composition according to the invention is also provided. A non-human animal according to the invention is especially useful for screening, detection and/or identification of candidate compounds capable of inhibiting or decreasing expression, amount and/or activity of miRNA-30 and/or miRNA-133. Such non-human test animal is also especially useful for screening, detection and/or identification of candidate compounds capable of decreasing interaction of CTGF mRNA with miRNA-30 and/or miRNA-133. Hence, a non-human test animal according to the invention is especially useful for screening, detection and/or identification of candidate compounds capable of inducing or enhancing fibrosis. If a candidate compound appears to have this property, it is recommended to avoid administration to human subjects.

In yet another embodiment, a method is provided for screening, detection and/or identification of candidate compounds capable of counteracting, treating, diminishing, delaying and/or preventing fibrosis, preferably pulmonary fibrosis, liver fibrosis and/or renal fibrosis, more preferably cardiac fibrosis. In this embodiment, candidate compounds are typically screened for their potential capabilities of increasing the expression, amount and/or activity of miRNA-30 and/or miRNA-133, or increasing the interaction of CTGF mRNA with miRNA-30 and/or miRNA-133, or with a functional part or derivative thereof. If a candidate compound appears to have this property, it is capable of counteracting or preventing fibrosis. Further provided is therefore a method for determining whether a candidate compound is capable of counteracting, treating, diminishing, delaying and/or preventing fibrosis, preferably cardiac fibrosis, the method comprising:
- contacting said candidate compound with a cell, preferably with a cardiac cell, and
- determining whether said candidate compound is capable of increasing the expression, amount and/or activity of miRNA-30 and/or miRNA-133 in said cell.

In one preferred embodiment, a candidate compound is contacted with a cell according to the present invention wherein expression of miRNA-30 and/or miRNA-133 is diminished, so that the effects of candidate compounds upon miRNA-30 and/or miRNA-133 are more clearly visible. It is, however, also possible to use any cell or non-human animal currently known in the art in a screening method according to the invention. In one embodiment, a non-human animal according to the invention is used.

In another embodiment, candidate compounds are screened for their capability of increasing interaction of CTGF mRNA with miRNA-30 and/or miRNA-133, or with a functional part or derivative thereof. If a candidate compound appears to have this property, it is capable of counteracting or preventing fibrosis. Further provided is therefore a method for determining whether a candidate compound is capable of counteracting, treating, diminishing, delaying and/or preventing fibrosis, preferably cardiac fibrosis, the method comprising:
- contacting said candidate compound with a cell, preferably with a cardiac cell, and
- determining whether said candidate compound is capable of increasing interaction of CTGF mRNA with miRNA-30 and/or miRNA-133, or with a functional part or derivative thereof, within said cell.

The invention is further explained in the following examples. These examples do not limit the scope of the invention, but merely serve to clarify the invention.

### Examples

### Example 1

Here we provide several lines of evidence to show that CTGF is importantly regulated by two miRNAs; miR-133 and miR-30. First, the expression of both miRNAs is inversely related to the amount of CTGF in two rodent models of heart disease and in human pathological LVH. Second, in cultured cardiomyocytes and fibroblasts, knock-down of these miRNAs increase CTGF levels. Third, overexpression of miR-133 or miR-30c decrease CTGF levels, which is accompanied by decreased production of collagens. Fourth, we show that CTGF is a direct target of these miRNAs, as they directly interact with the 3'-UTR of CTGF. Taken together our results show that miR-133 and miR-30 importantly limit the production of CTGF. We also provide evidence that the decrease of these two miRNAs in pathological Left Ventricular Hypertrophy (LVH) allows CTGF levels to increase, which contributes to collagen synthesis. In conclusion, our results show that both miR-133 and miR-30 directly down-regulate CTGF, a key pro-fibrotic protein, and thereby establish an important role for these miRNAs in the control of structural changes in the ECM of the myocardium.

### METHODS

### Cell culture

Rat ventricular myocytes (RCMs) and fibroblasts (RCFs) were isolated by enzymatic digestion of 1- to 2 day old neonatal rats as described previously¹⁷.

### Transduction and transfection of cultured RCMs and RCFs

Lentivirus for overexpression of miRNAs of interest were generated using pcDH1 expression vectors (pcDH1 Lentivectors, System Biosciences). For lentiviral miRNA overexpression 2,5 X 10⁵ RCMs per well were plated on gelatinized six-well plates and cultured overnight in DMEM/M199 (4:1) media (supplemented with 10% horse serum, 5% newborn calf serum, glucose, gentamycin and AraC) and 1,5 X 10⁵ RCFs per well were plated in six well plates and cultured overnight in DMEM (supplemented with 10% foetal bovine serum and gentamycin). The next day, the RCMs and RCFs were placed on low serum media and transduced with miR133b, miR-30c or the control-miRNA virus, facilitated by sequa-brene (Sigma Aldrich). After puromycin selection (3µg/ml), infection efficiencies were above 80%. After 5 days of culturing, cells were harvested for RNA or protein isolation. For miRNA inhibitor or mimic transfection 1 X 10⁶ RCMs, and 1,5 X 10⁵ RCFs per well were plated on six-well plates and cultured overnight (see above). Then the cells were placed on low serum for 24 hr. The next day they were placed on serum-free media without antibiotics for 4 hr. Cells were then transfected with 100 nM mi*RIDIAN* rno-miR-133b miRNA inhibitor (I-320198-00), mi*RIDIAN* rno-miR-30c miRNA inhibitor (I-320075-03), *miRIDIAN* miRNA inhibitor Negative Control #2 (IN-002000-01), mi*RIDIAN* rno-miR-30c miRNA mimic (C-320075-03) and mi*RIDIAN* miRNA mimic negative control #2 (CN-002000-01) (all obtained from Dharmacon) using Lipofectamin 2000 (11668, Invitrogen) according to manufacturers protocol. After 48 hr of culturing, cells were isolated for RNA or protein isolation.

### Real time PCR

Total RNA was isolated from tissues and cells with the *mi*RVana™ miRNA isolation kit (Ambion) according to manufacturers' protocol. Total and miRNA specific cDNA was generated with iScript™ cDNA synthesis kit (Biorad) and *mir*Vana™ qRT-PCR miR-133 and miR-30c Primer set (30033 and 30144, Ambion).

### Western blotting

Protein was isolated after grinding frozen heart tissue with RIPA buffer. Cells were lysed with standard sample buffer, sheared through a 23G needle and boiled for 5 min. Primary antibodies for the detection of CTGF and GAPDH were resp. ab6992 (Abcam) and 6C5 (RDI).

### Plasmid construction

*pCDH1-miR133b:* rno-miR-133b precursor DNA was PCR-amplified from rat genomic DNA with the primers: 5'-ACTGGAATTCGTGAGTGGTTAGTCAGGGTAATG-3' and 5'-ACTGGCGGCCGCCTATGATCTCTTGTGAACCTGGG-3'. This fragment (375 bp) was cloned into the pCDH1-MCS1-EF1-Puro vector (System Biosciences) under the control of a CMV promoter.

*pCDH1-miR30c:* rno-miR-30c-1 precursor DNA was PCR-amplified from rat genomic DNA with the primers: 5'-AGCTGAATTCTTTTACTCAGCCCATGTGGTCG-3' and 5'-AGCTGCGGCCGCGCATTCTGTCCGATCTGTTGGGTG-3'. This fragment (263 bp) was cloned into the pCDH1-MCS1-EF1-Puro vector (System Biosciences) under the control of a CMV promoter.

*pCDH1-control-miRNA:* this vector is based on the pcDNA™6.2-GW/miR-neg control plasmid (Invitrogen) which contains an insert that can form a hairpin structure that is processed into mature miRNA but is predicted not to target any known vertebrate gene.

Luciferase reporter plasmid was constructed by PCR-amplifying the 3'UTR from rat CTGF with the primers: 5'-GAGTAAGGGACACGAACT-3' and 5'-GAAAGGTGCAAACATGTAAC-3'. This 1023 bp fragment was cloned into the pMIR-REPORT™ Luciferase vector (Ambion) downstream of the luciferase coding region, creating luciferase-CTGF 3'UTR. The complete miR-133 binding site was deleted by PCR-based mutagenesis creating the luciferase CTGF-3'UTR mutant. Mef2C-flag reporter plasmids were constructed by cloning the Mef2C open reading frame (flag-tagged) behind the CMV-promoter of the pcDNA3.1 vector (Invitrogen). Next, we subcloned the rat CTGF 3'UTR and rat CTGF 3'UTR mutant downstream of the Mef2C open reading frame, creating Mef2C-flag-CTGF-3'UTR and Mef2C-flag-CTGF-3'UTR mutant reporter constructs. All generated constructs were sequence verified.

### miRNA reporter assays

For luciferase assays we transiently transfected (GeneJammer, Stratagene) 6-galactosidase control plasmid (50 ng), wild-type or mutant luciferase-CTGF-3'UTR reporter (25 ng) and pCDH1-miR-133b or pCDH1-miR-30c overexpressing construct (25 ng) into 40-50% confluent COS1 cells, grown in a 24-well plate. At 48 hr post transfection, cells were lysed and assayed for luciferase activity. Beta-galactosidase was assayed to normalize luciferase results for cell densities and transfection efficiency. For the western blot-based miRNA reporter assay, we transfected wild-type or mutant flag Mef2C-CTGF-3'UTR (25 ng), miR-133b or miR-30c overexpression construct (50, 100 and 200 ng), and empty pCDH1 as filler into COS1 cells as described above. At 48 hr post transfection, cells were harvested for Western blot using flag antibody (Sigma).

### Animal models

The homozygous Ren2 rat is a model of hypertension induced heart failure. Here the mouse renin-2 gene has been introduced into the rat genome, causing activation of the renin-angiotensin system and resulting in cardiac hypertrophy by the age of 8 weeks, and heart failure before the age of 18 weeks^{18, 19}. Cardiac hypertrophy develops invariably. Some of these rats rapidly progress to heart failure, whereas other similarly hypertensive littermates remain compensated²⁰. Heart function of 10-wk-old Ren-2 and Sprague-Dawley rats was monitored by serial echocardiography at 10, 12, 15, 16, 18, 19 and 21 wk of age and animals were sacrificed at 15-18 wk upon clinical signs of heart failure (HF-prone rats) or at 21 wk when clinical signs of failure had not appeared (compensated rats). Another group of Ren-2 and Sprague-Dawley (controls) rats were monitored and euthanized at 10 and 16 wks of age when clinical signs of failure had not appeared.

Male and female C57Black6 mice were subjected to transverse aortic binding or sham surgery as has been previously described²¹.

### Patients

Nine patients with isolated aortic stenosis undergoing valve replacement surgery were included in the study. Four patients undergoing coronary artery bypass grafting (CABG) were included as non-hypertrophic controls²². CABG patients had normal ejection fraction, no unstable angina and no history of myocardial infarction or LVH²². During open-heart surgery, but before extra-corporal circulation, two to three transmural needle biopsies were taken from the anterior LV, and snap-frozen in liquid nitrogen for RNA isolation. The institutional ethics committee of the University Hospital Maastricht approved the study, and all patients gave informed consent.

### In Situ Hybridization

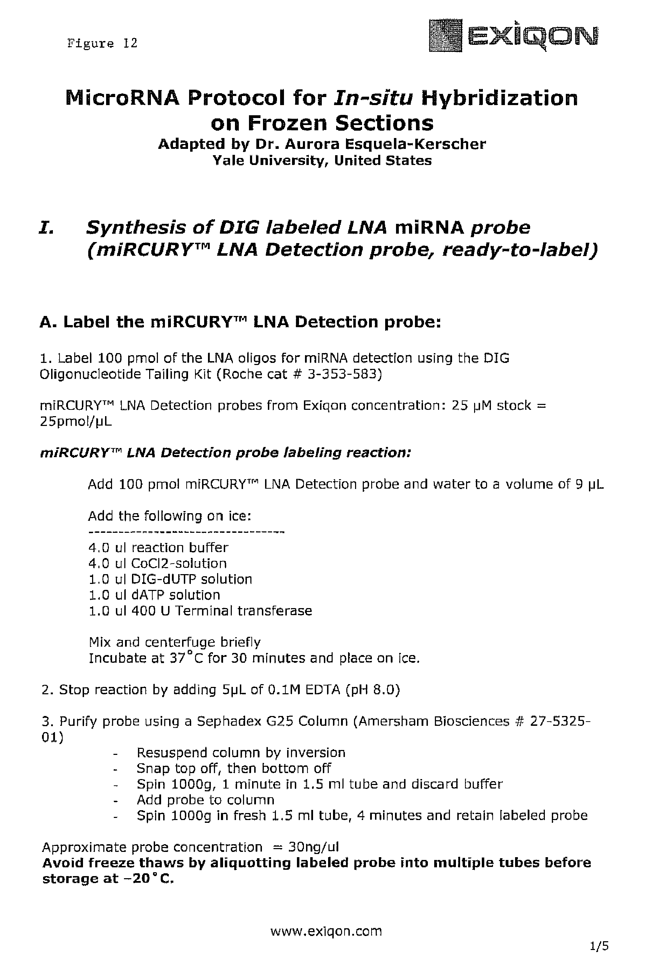

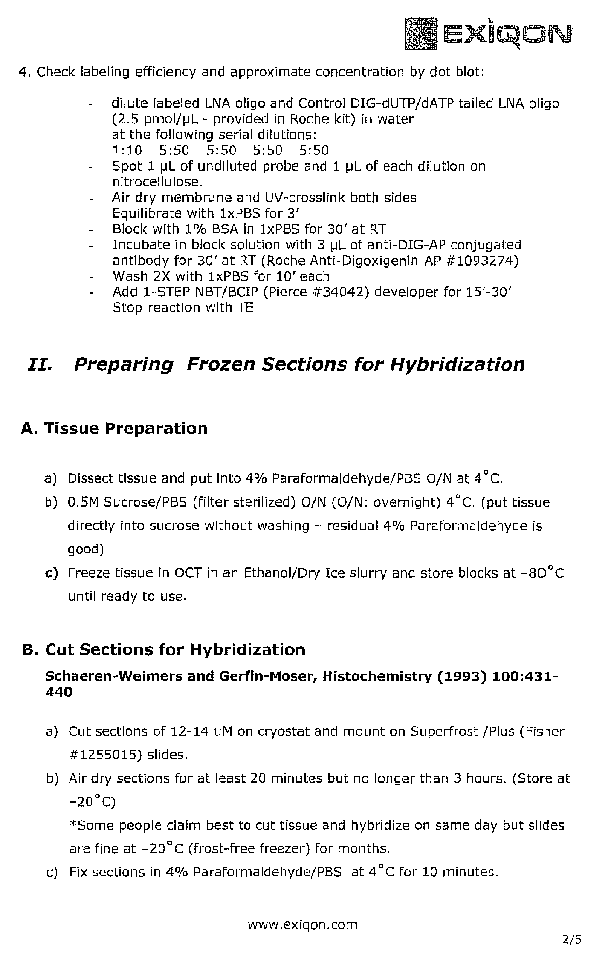

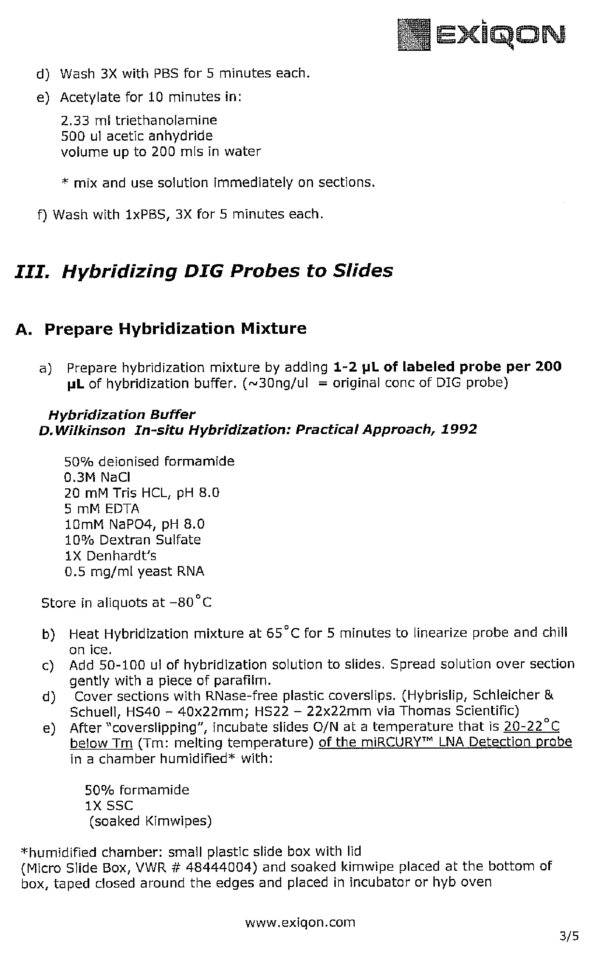

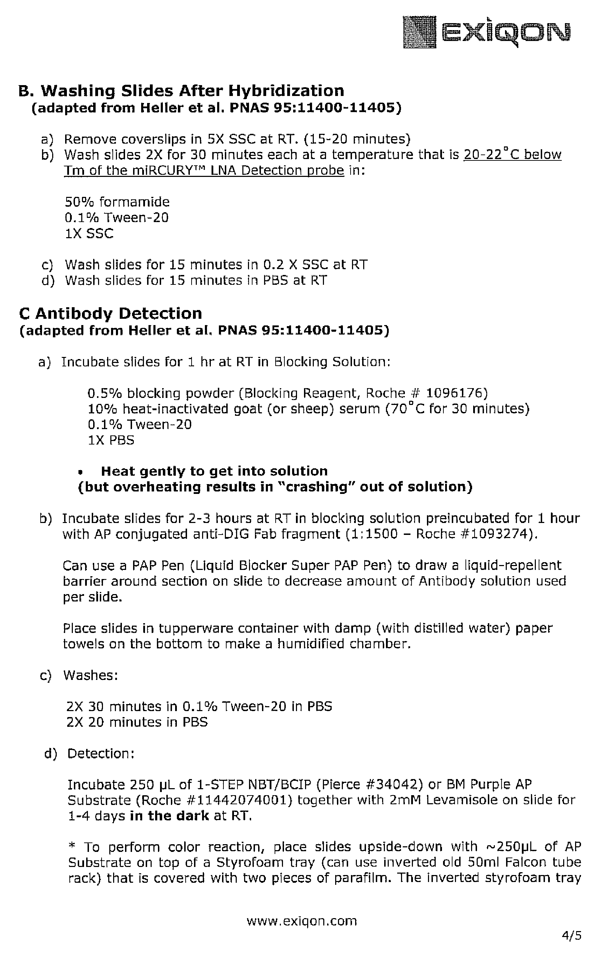

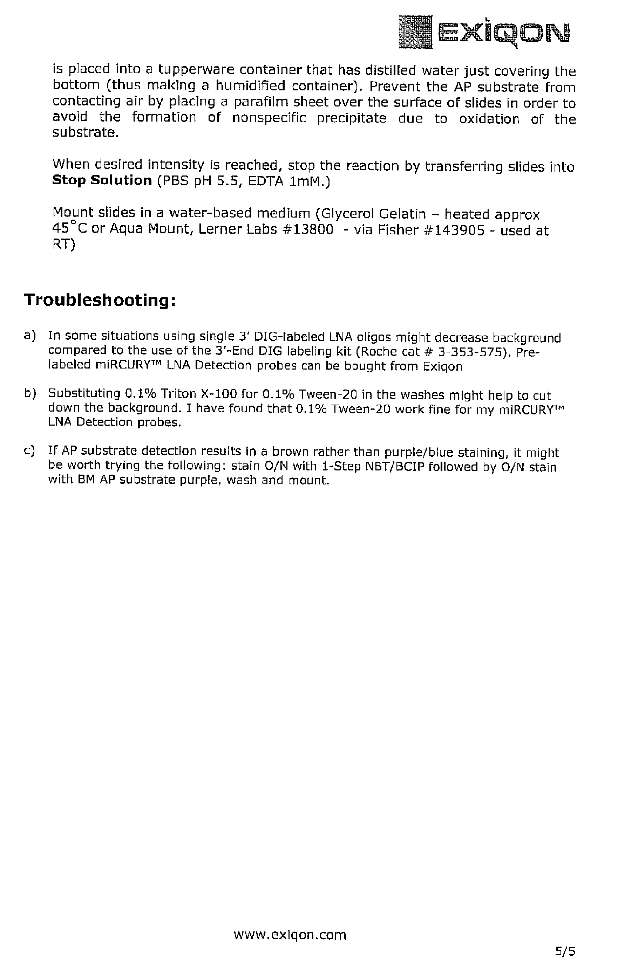

LNA hybridization probes complementary to human mature miR-133b (38033-05) and a scrambled probe (99001-05) with 3'-digoxigenin (DIG) conjugate were purchased from Exiqon (Vedbaek, Denmark). MiRNA *in situ* hybridization was performed as described at www.exiqon.com/insitu. This protocol is shown in Figure 12.

### RESULTS

### CTGF expression is increased in pathological LVH

The importance of CTGF as a mediator of tissue fibrosis has been recognized in many different tissues and forms of pathology^{3, 6-9}. Also in the heart a close correlation has been observed with cardiac CTGF levels and progressive fibrosis^{8, 10,23}. In line with these findings we show that CTGF levels are substantially increased in two established rodent models of cardiac hypertrophy. In the first model, the Ren2 rat model of hypertension-induced LVH, rats develop severe hypertension at 8 weeks of age, and severe end-organ damage, including heart failure and fibrosis. With similar levels of LVH, 50% of the littermates either quickly progress to heart failure while the other 50% stay compensated for prolonged periods of time. By using sequential tissue sampling in these ren2 rats we show that CTGF mRNA and protein is upregulated already in the early phases of hypertrophy (Fig. 1A and 1B). In a second model we subjected mice to thoracic aortic banding (TAC), which induces hypertrophy by increased afterload of the heart and is accompanied by significant fibrosis (not shown). Also in these hearts we observed an increased expression of CTGF (Fig. 1A and B). To determine whether CTGF expression is also increased in human heart disease, we conducted real-time PCR on cardiac biopsies of patients with aortic valve stenosis (ventricular hypertrophy) and CABG patients (non-hypertrophic). We found that in the hypertrophic human hearts CTGF expression was induced at least 5-fold compared to the control ventricles (Fig. 1A, right panel).

### Connective Tissue Growth Factor harbors phylogenetically conserved

To explore whether miRNAs could regulate CTGF we undertook a bioinformatic approach using miRanda software (www.microrna.org) and found approximately ten miRNAs that could potentially target CTGF mRNA. The two most notable miRNAs were miRNA-133 and miR-30c, since those miRNAs were significantly down-regulated in miRNA arrays performed on hypertrophic and failing hearts of the hypertensive Ren2 rat model (not shown). Alignment of the 3'-UTR of CTGF among a wide range of species (human, rat, mouse, chicken and xenopus tropicalis) revealed that the predicted binding sites for miR-133 and miR-30c are highly conserved during evolution, suggesting the potential importance of these binding sites in CTGF (Fig. 1C).

### MiR-133 and miR-30c are down-regulated in pathological LV hypertrophy

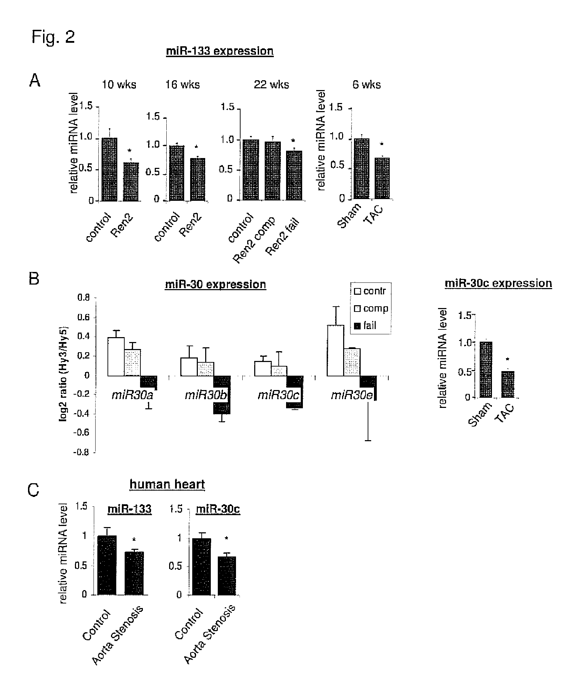

MiR-133 has recently been demonstrated to play a central role in cardiac hypertrophy and several groups have shown that the expression of miR-133 is significantly down-regulated in human and mouse hypertrophic hearts^{14-16, 24}. In Fig. 2A we extend the described loss of mature miR-133 in hypertrophic hearts, in our two rodent models of LV hypertrophy and heart failure. Already very early in the course of pathological LV hypertrophy, in 10 weeks old Ren2 rats, we observed a significant down-regulation in the expression of miR-133. At later stages, during the transition to heart failure, mature miR-133 levels continued to be repressed compared to the Sprague-Dawley controls. Also in TAC hearts we observed a loss of miR-133 when compared to the sham operated controls (Fig. 2A, right panel).

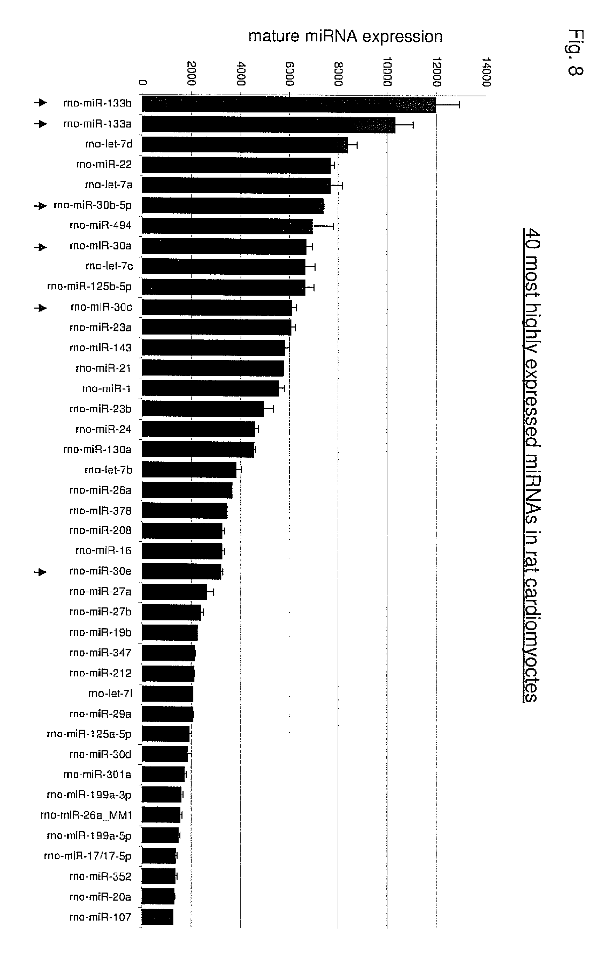

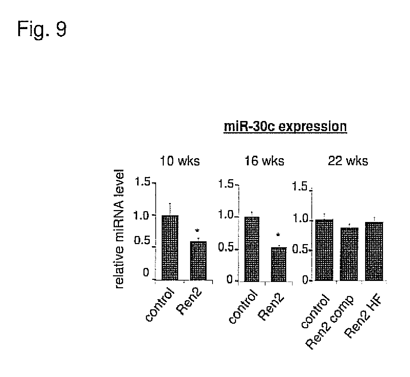

Several miR-30 family members are abundantly expressed in cardiomyocytes (Fig. 8). Strikingly, we observed a robust down-regulation of multiple members of the miR-30 family in the LV of failing Ren-2 hearts (Fig.2B and Fig. 9). MiR-30c was also significantly down-regulated in the diseased LV of the banded mice (Fig. 2B) and in cardiac biopsies from LVH patients (Fig. 2C). Together these results show reduced miR-133 and miR-30c expression in several forms of pressure loading-induced LV hypertrophy and raise the intriguing insight that loss of miR-133 and miR-30 actually cause accumulation of CTGF protein and thereby contribute to tissue fibrosis in the diseased heart.

### MiR-133 is expressed mainly in cardiomyocytes

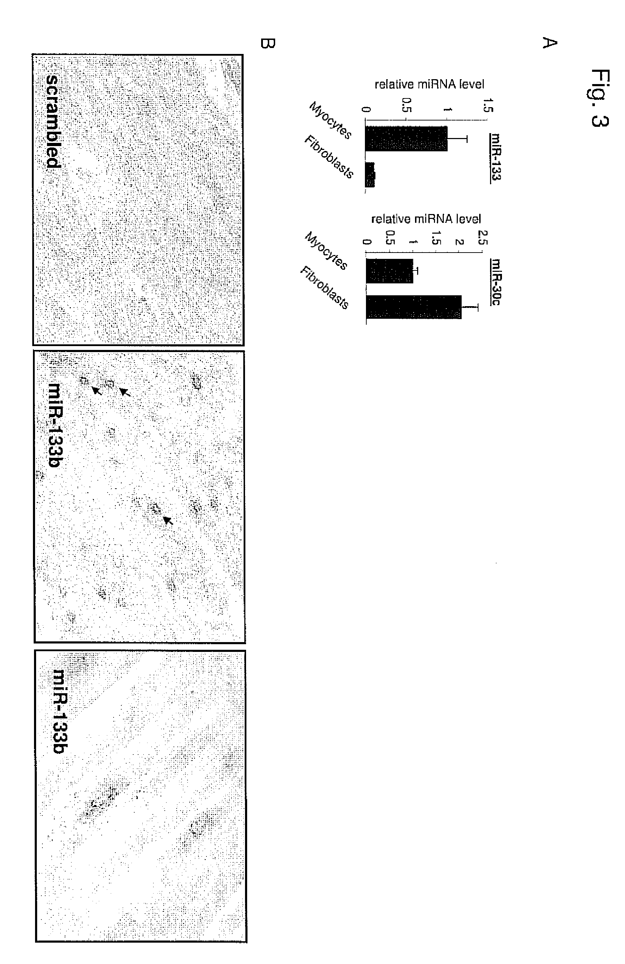

Most miRNAs, including miR-133, are expressed in a tissue-specific manner²⁵. While it is generally accepted that miR-133 and miR-30c are expressed in the adult heart, the exact cell type responsible for the production of those miRNAs has not been described. To examine the cell types responsible for expression of miR-133 and miR-30c in the heart, we performed real-time PCR on equal amounts of RNA isolated from cultured rat cardiac myocytes and cultured rat cardiac fibroblasts and normalized the expression for GAPDH levels. As shown in Figure 3A, we detected approximately 15-fold higher miR-133 levels in myocytes than fibroblasts. This indicates that miR-133 is highly specific for cardiac myocytes. Next, we performed *in situ* hybridizations using LNA probes for mature miR-133 in frozen sections of normal rat hearts. As shown in figure 3B mature miR-133 was detected at high levels in the rat heart, while a scrambled probe serving as a negative control showed no signal. High-powered magnification (Fig. 3B, right panel) revealed that mature miR-133 is concentrated in cytoplasmic foci in the proximity of the nucleus. The presence of miR-133 in those so called P-bodies, the cellular compartment where miRNA and miRNA-repressed mRNAs are located in mammalian cells²⁶, shows that miR-133 is actively involved in post-transcriptional regulation in cardiomyocytes. Notably, no miR-133 signal was detected in vascular structures, which further shows that cardiomyocytes exclusively express this miRNA in the heart.

Real-time PCR demonstrated that mature miR30c levels were 2-fold higher in cardiac fibroblasts compared to cardiac myocytes (Fig. 3A), illustrating that the two cell types of the heart known to express CTGF, also have the potential to post-transcripionally regulate CTGF levels by miRNAs.

### MiR-133 and miR-30c regulate CTGF mRNA and protein levels

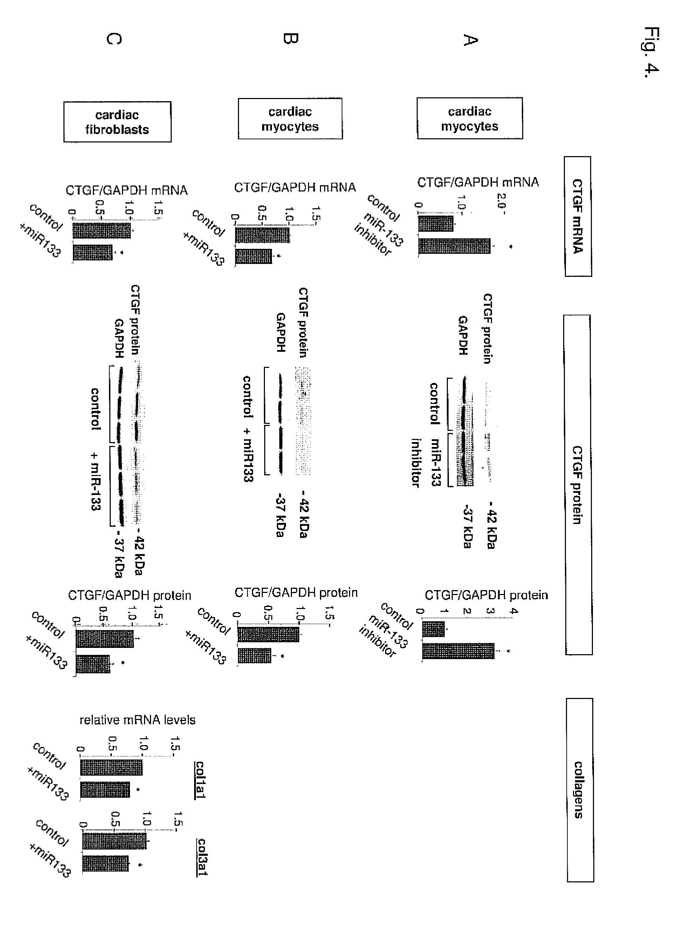

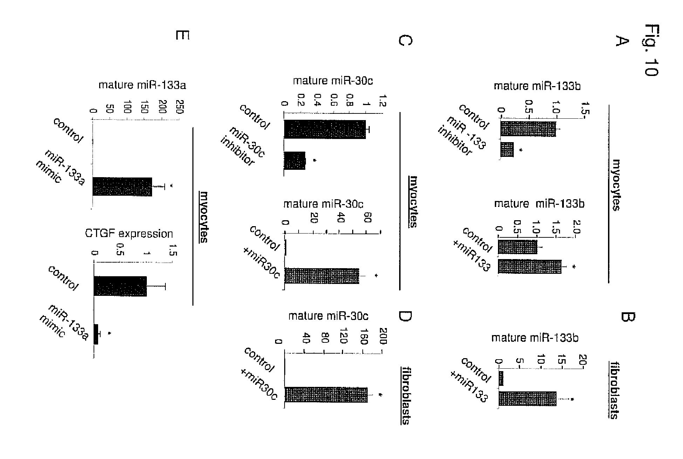

We then performed a series of functional studies to determine the role of miR-133 and miR-30 in the regulation of CTGF. To investigate whether miR-133 regulates endogenous CTGF levels, we transfected cultured cardiac myocytes with RNA oligonucleotides, complementary to mature miR-133b (miR-133 inhibitor) or a scrambled sequence that served as control (Fig. 4A). Successful knock-down of miRNAs in these cells was confirmed and shown in figure 10. These experiments demonstrated that specific knock-down of miR-133b robustly induce CTGF mRNA and protein levels. Next, we tested whether miR-133 overexpression is sufficient to repress CTGF levels. For this purpose, we overexpressed miR-133b by using a lentivirus in cardiac myocytes and fibroblasts. Real-time PCR showed >1000 fold increase ofpre-miR-133 (not shown), while mature miR-133 was only induced 1,5-2.0 fold in myocytes (Fig. 10A) and 14-fold in fibroblasts (Fig. 10B). The observed difference between pri- and mature miRNA levels in cardiac myocytes suggests that processing ofpre-miR-133 into mature miR-133 has a low efficiency in these cells. Nevertheless, the 1,5-2.0 fold increase of mature miR-133 was sufficient to blunt endogenous CTGF mRNA and protein levels substantially in cultured cardiac myocytes (Fig. 4B) and fibroblasts (Fig. 4C). The miR-133-induced down-regulation of CTGF in turn resulted in a significant decrease in pro-fibrotic signaling as shown by reduced production of collagen type I and III mRNA in cardiac fibroblasts (Fig. 4C). That miR-133a is also capable of regulating CTGF mRNA is demonstrated in an experiment where miR-133a was overexpressed in cultured cardiomyocytes using double-stranded RNA oligos comprising the mature miR-133a sequence (miR-133a mimic). Robust down-regulation of CTGF in this experiment is shown in figure 10E, and led us to conclude that both miR-133a and miR-133b are potent regulators of CTGF expression.

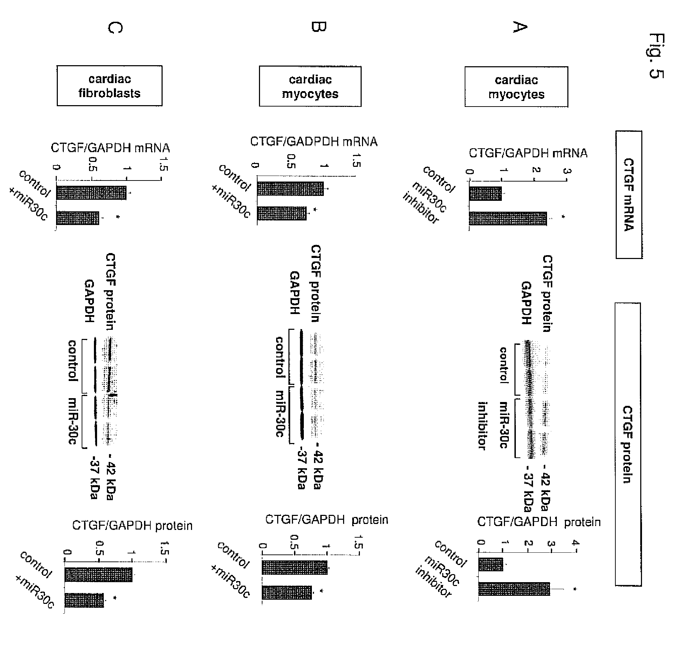

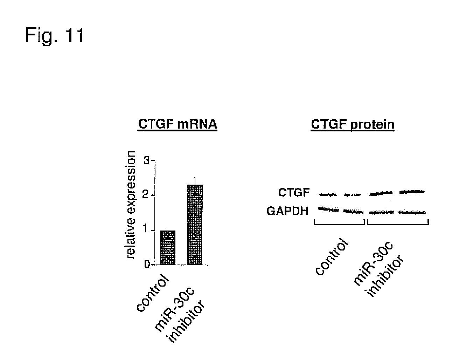

Also miR-30c appeared a potent regulator of CTGF expression: silencing miR-30c in cultured cardiac myocytes using miR-inhibitors induced CTGF mRNA and protein levels about 2-3 fold (Fig. 5A and Fig. 10C). Also in fibroblasts CTGF protein levels largely depended on endogenous miR-30, as demonstrated by knock-down of miR-30c transfections in these cells (Fig. 11). To test whether overexpression of miR-30c is sufficient to blunt CTGF expression we transfected cardiomyocytes and fibroblasts with double-stranded RNA oligos comprising the mature miR-30c sequence (miR-30 mimic) or a non-targeting control miRNA (Fig 10C). In line with the knock-down experiments, miR-30c mimic was sufficient to induce down-regulation of CTGF mRNA and protein in both cardiac myocytes and fibroblasts (Fig. 5 B and C, respectively). Combined knock-down of miR-133 and miR-30 did not result in a synergistic or additive increase in CTGF expression (data not shown). We attribute this to the fact that the predicted miRNA binding sites in the 3'-UTR of CTGF for miR-133 and miR-30 display considerable overlap (Fig. 1C) and therefore may spatially hinder each other from binding the 3'-UTR simultaneously. Moreover, occupancy of CTGF mRNA by both miRNAs simultaneously is of limited physiological relevance since the miR-133 is expressed exclusively in cardiac myocytes while miR-30c is expressed predominantly in fibroblasts.

### CTGF is a direct target of miR-133 and miR-30

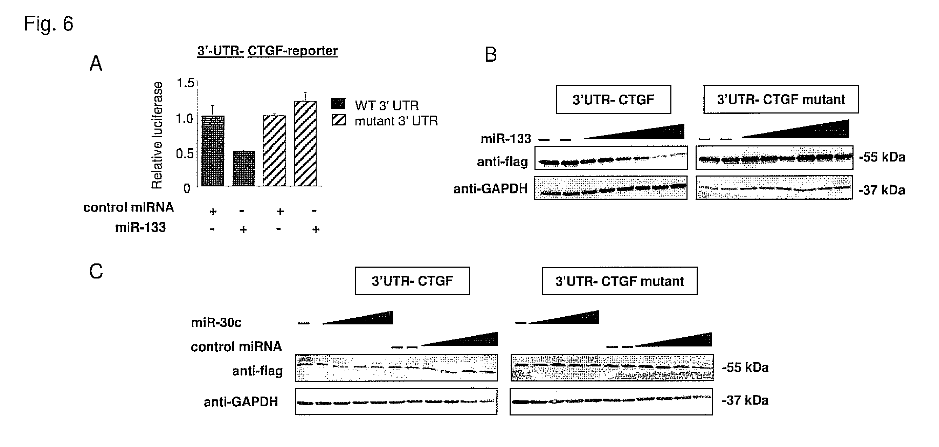

The above shows that CTGF is regulated by miR-133 and miR-30c in pathological LVH. However, the miRNA effects we observed on CTGF expression could be indirect. To test whether the putative miR-133 target sequence in CTGF 3'UTR (Fig. 1C) directly regulates protein levels of CTGF, we inserted the full-length 3'UTR of the CTGF transcript into a luciferase expression plasmid, which we then transfected into COS1 cells. Co-transfection of this luciferase reporter with miR-133, but not a control miRNA resulted in a significant decrease in luciferase activity. Deletion of the miRNA binding site, only 28 nucleotides within the 1023 nt UTR, abrogated the repressive effect of miR-133 on luciferase activity (Fig 6A), showing that the expression of CTGF is extremely sensitive to the miR-binding sequence within the 3'-UTR. MiR-133 also dose-dependently inhibited the synthesis of Flag-tagged-MEF2C expression cassette linked to the CTGF 3'UTR binding sequence, but not the mutant CTGF 3'-UTR, in which the miR-133 target site was deleted (Fig. 6B).

To test whether the putative miR-30 target sequence in CTGF 3'UTR could mediate translational repression we performed the same type of western blot based reporter assay as described above. Co-transfection of the expression vectors containing miR-30c and Flag-MEF2C-3'-UTR-CTGF construct shows less flag-tagged protein compared to the scrambled miRNA, but not when the miR-30c binding site is deleted from the construct (Fig. 6C). Overall, these results show that miR-133 and miR-30 directly influence CTGF protein levels through specific binding to its 3'UTR.

### Example 2

Transgenic mice overexpressing miR-30 specifically in cardiac myocytes are generated. Hearts of these mice are evaluated using echocardiography and after sacrifice using histological techniques. Besides these baseline studies, the hearts of the miR-30 transgenes are also stressed using transaortic banding (TAC) and placement of osmotic minipumps to precisely evaluate the role of miR-30 in fibrosis of the heart. These mice will produce less CTGF protein and therefore will develop less fibrosis in their hearts.

In parallel to these gain-of-function studies, loss-of-function studies are also performed in a transgenic mouse model. For this purpose a decoy construct is overexpressed specifically in cardiomyocytes. This decoy mRNA contains multiple binding sites for miR30, and when overexpressed it depletes the cytosol of cardiomyocytes of miR30. This results in reduced binding of miR30 to CTGF mRNA and thus increased CTGF levels and more fibrosis in the hearts of these mice. Hearts of these mice are evaluated using echocardiography and histology.

Together these studies demonstrate that miR30 regulates CTGF levels and fibrosis *in vivo.*

### Brief description of the drawings

**Fig. 1** **CTGF expression is elevated in pathological LV hypertrophy**
   **A)** CTGF mRNA is elevated in hearts of 10 week old Ren-2 rats (n=9), 16 week old Ren-2 rats (n=7) and failing Ren-2 rats (n=6) and to a lesser extent in compensated Ren-2 rats (n=5), as analyzed by real-time PCR. CTGF mRNA is also elevated after transverse aortic constriction (TAC, n=5) compared to sham operated mice (n=10) and in cardiac biopsies of patients with LV hypertrophy (aorta stenosis, n=9) compared to 'healthy' human myocardium (CABG, n=4). CTGF expression was normalized for expression of the housekeeping gene PGK-1 and presented as mean ± sem. *: p<0.05 compared to control or sham heart, ^{$} p< 0.05 compared to compensated Ren-2 hearts. B) Western blot for CTGF and GAPDH in hearts of 16 week old Ren-2 rats (n=4/group) and TAC mice (n=4/group). C) Sequence alignment between miR-133 and miR-30c and the 3' UTR of CTGF in several species.
**Fig. 2****. Loss of mature miR-133 and miR-30 in cardiac hypertrophy and failure**
   **A)** Mature miR-133 is down-regulated in the hearts of 10 week old Ren-2 rats (n=9), 16 week old Ren-2 rats (n=6), in failing Ren2 hearts (n=6) and in hypertrophic mouse hearts (TAC surgery, n=5), as detected by real-time PCR. B) Loss of expression of multiple members of the miR-30 family in failing rat hearts (detected by miRNA arrays, Exiqon) and in hypertrophic mouse hearts (TAC surgery, n=5) C) Real-time PCR analysis on cardiac biopsies of patients with LV hypertrophy (aorta stenosis, n=9) compared to 'healthy' human myocardium (CABG, n=4) for mature miR-133 and mature miR-30c. MiRNA expression was normalized for expression of PGK1 and presented as mean ± sem. *: p<0.05 compared to control or sham heart.
**Fig. 3****. miR-133 is expressed in cardiac myocytes and miR-30c mainly in fibroblasts**
   **A)** In situ hybridization on frozen sections of healthy rat heart with Dig labeled LNA probe for miRNA-133 and a scrambled probe. High-powered magnification (right panel) shows mature miR-133 in cytoplasmic foci in the proximity of the nucleus (P-bodies). B) Real-time PCR detection of mature miR-133 and miR-30c in cultured neonatal rat cardiac myocytes and fibroblasts corrected for GAPDH expression.
**Fig. 4****. miR-133 regulates CTGF mRNA and protein levels**
   Knock-down (A) and overexpression experiments (B and C) of miR-133 in cultured myocytes and fibroblasts. For degree of miR-133 knock-down and overexpression, see figure 10. **A**) Knock-down of miR-133, by transfection of miR-133 inhibitor in cultured cardiac myocytes, enhances CTGF mRNA and protein levels, as measured by real-time PCR and western blot, respectively. Quantification of protein bands reveals a 3-fold increase of CTGF protein after knock-down of miR-133 (right figure). Lentiviral overexpression of miR-133 represses CTGF mRNA and protein levels in cardiac myocytes (B) and fibroblasts (C) to similar extents. Loss of CTGF protein affects fibrotic gene expression as evidenced by a concomitant decrease in procollagen type 1 and type 3 expression in the miR-133 treated fibroblasts (C, right panel). *: p<0.05 compared to control treated cells.
**Fig. 5****. miR-30c regulates CTGF mRNA and protein levels**
   Knock-down (A) and overexpression experiments (B and C) of miR-30c in cultured myocytes and fibroblasts. Degree of miR-30c knock-down and overexpression is shown in figure 10. **A)** miR-30c inhibitor enhances CTGF mRNA and protein levels in cultured cardiac myocytes, as analyzed by real-time PCR and western blot, respectively. Quantification of protein bands is shown in right panel. **B)** Overexpression of miR30c results in reduced CTGF mRNA and protein in cultured cardiac myocytes and **C)** fibroblasts. *: p<0.05 compared to control treated cells.
Fig. 6. CTGF is a direct target of miR-133 and miR-30c
   **(A)** Cos cells were transfected with CTGF-3'-UTR luciferase construct together with expression plasmids for miR-133 or a scrambled miRNA (control miRNA). miR-133 overexpression decreased luciferase activity of the wild-type but not the mutant 3'-UTR where the miR-133 binding site was deleted. **(B)** Dose-dependent inhibition of flag-CTGF-3'-UTR by miR-133 as demonstrated by western blot with flag antibody. **C)** Western blot on Cos cells transfected with flag-CTGF-3'-UTR and increasing concentrations of miR-30c or a control (scrambled) miRNA.

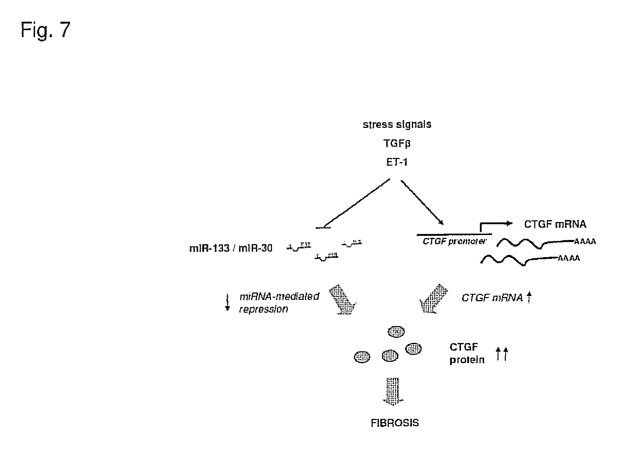

**Fig. 7****. A model for the regulation of CTGF expression in the diseased heart.**
   Stress signals in the heart induce CTGF mRNA expression directly through TGF6-and endothelin-1 (ET-1) responsive elements in the promoter. At the same time, stress signals diminish miR-133 and miR-30 expression. As a direct target of miR-133 and miR-30, CTGF protein levels accumulate even further upon loss of miRNA-mediated repression. Modulation of miR-133 and miR-30c expression may be of therapeutic value to control cardiac fibrosis.
**Fig. 8****. miRNA Expression profiling in cultured neonatal rat cardiomyocytes**
   MiRNA arrays (Exiqon) were performed on RNA isolated from cultured rat cardiomyocytes. miRNAs are ranked from left to right on the basis of their expression level (spot intensities). Arrow heads indicate expression levels of miR-133 and miR-30 family members.
**Fig. 9****. Loss of mature miR-30c in ren2 rats**
   Real-time PCR for mature miR-30c in ren2 rats at several ages, demonstrating the most robust loss of miR-30c in the early stages of hyperrophy. MiRNA expression was normalized for expression of PGK1 and presented as mean ± sem. *: p<0.05 compared to control or sham heart. n<5 per group.
**Fig. 10****. Levels of knock-down and overexpression of miRNAs in primary cells.**
   **A)** Real-time PCR for mature miR-133b in cardiac myocytes transfected with miR-133b inhibitor (left panel) or after lentiviral overexpression of miR-133b (right panel).
   **B)** robust overexpression of miR-133b in isolated rat cardiac fibroblasts after transduction with a lentivirus containing pri-miR-133b. **C)** Real-time PCR for mature miR-30c in cardiac myocytes transfected with miRNA inhibitor (left panel) or mimic (right panel) for miR-30c. **D)** Successful overexpression of miR-30c in primary cardiac fibroblasts, detected by real-time PCR for mature miR-30c primers. Mature miRNA expression is normalized for GAPDH expression in the same samples. Data presented as mean ± SEM. * p < 0.05 compared to control (scrambled antagomir or lentivirus).
   **E)** Overexpression of miR-133a (left panel, *:p<0.05), using miR-133a mimic resulted in significant downreguation of CTGF in cultured cardiomyocytes (right panel, p<0.05). Expression of miR-133a and CTGF is normalized for the expression of the housekeeping gene GAPDH.
**Fig. 11****. Loss of miR-30c in fibroblasts induces CTGF mRNA and protein.**
   Left panel: Real-time PCR for CTGF in isolated fibroblasts transfected with a control or miR-30 directed antagomir. Right panel: western blot revealing increased CTGF protein levels after silencing miR-30c in isolated cardiac fibroblasts using antagomirs. As a control we used a scrambled miRNA.
**Fig. 12****. MicroRNA protocol for in-situ hybridization on frozen sections.**

### References

**1.** Bornstein P, Sage EH. Matricellular proteins: extracellular modulators of cell function. Curr Opin Cell Biol. Oct 2002;14(5):608-616.
**2.** McMullen JR, Jennings GL. Differences between pathological and physiological cardiac hypertrophy: novel therapeutic strategies to treat heart failure. Clinical and experimental pharmacology & physiology. Apr 2007;34(4):255-262.
**3**. Shi-Wen X, Leask A, Abraham D. Regulation and function of connective tissue growth factor/CCN2 in tissue repair, scarring and fibrosis. Cytokine & growth factor reviews. Apr 2008;19(2):133-144.
**4.** Moussad EE, Brigstock DR. Connective tissue growth factor: what's in a name? Molecular genetics and metabolism. Sep-Oct 2000;71(1-2):276-292.
**5.** Perbal B. CCN proteins: multifunctional signalling regulators. Lancet. Jan 3 2004;363(9402):62-64.
**6.** Chen MM, Lam A, Abraham JA, Schreiner GF, Joly AH. CTGF expression is induced by TGF- beta in cardiac fibroblasts and cardiac myocytes: a potential role in heart fibrosis. J Mol Cell Cardiol. Oct 2000;32(10):1805-1819.
**7.** Morikawa H, Tamori A, Nishiguchi S, Enomoto M, Habu D, Kawada N, Shiomi S. Expression of connective tissue growth factor in the human liver with idiopathic portal hypertension. Mol Med. May-Jun 2007;13(5-6):240-245.
**8.** Koitabashi N, Arai M, Kogure S, Niwano K, Watanabe A, Aoki Y, Maeno T, Nishida T, Kubota S, Takigawa M, Kurabayashi M. Increased connective tissue growth factor relative to brain natriuretic peptide as a determinant of myocardial fibrosis. Hypertension. May 2007;49(5):1120-1127.
**9**. Ivkovic S, Yoon BS, Popoff SN, Safadi FF, Libuda DE, Stephenson RC, Daluiski A, Lyons KM. Connective tissue growth factor coordinates chondrogenesis and angiogenesis during skeletal development. Development (Cambridge, England). Jun 2003;130(12):2779-2791.
**10.** Ohnishi H, Oka T, Kusachi S, Nakanishi T, Takeda K, Nakahama M, Doi M, Murakami T, Ninomiya Y, Takigawa M, Tsuji T. Increased expression of connective tissue growth factor in the infarct zone of experimentally induced myocardial infarction in rats. J Mol Cell Cardiol. Nov 1998;30(11):2411-2422.
**11**. Callis TE, Wang DZ. Taking microRNAs to heart. Trends in molecular medicine. Jun 2008;14(6):254-260.
**12.** Latronico MV, Catalucci D, Condorelli G. Emerging role of microRNAs in cardiovascular biology. Circ Res. Dec 7 2007;101(12):1225-1236.
**13.** van Rooij E, Olson EN. MicroRNAs: powerful new regulators of heart disease and provocative therapeutic targets. J Clin Invest. Sep 2007;117(9):2369-2376.
**14**. Care A, Catalucci D, Felicetti F, Bonci D, Addario A, Gallo P, Bang ML, Segnalini P, Gu Y, Dalton ND, Elia L, Latronico MV, Hoydal M, Autore C, Russo MA, Dorn GW, 2nd, Ellingsen O, Ruiz-Lozano P, Peterson KL, Croce CM, Peschle C, Condorelli G. MicroRNA-133 controls cardiac hypertrophy. Nat Med. May 2007;13(5):613-618.
**15.** Cheng Y, Ji R, Yue J, Yang J, Liu X, Chen H, Dean DB, Zhang C. MicroRNAs are aberrantly expressed in hypertrophic heart: do they play a role in cardiac hypertrophy? Am J Pathol. Jun 2007;170(6):1831-1840.
**16.** van Rooij E, Sutherland LB, Liu N, Williams AH, McAnally J, Gerard RD, Richardson JA, Olson EN. A signature pattern of stress-responsive microRNAs that can evoke cardiac hypertrophy and heart failure. Proc Natl Acad Sci U S A. Nov 28 2006;103(48):18255-18260.
**17.** De Windt LJ, Willemsen PH, Popping S, Van der Vusse GJ, Reneman RS, Van Bilsen M. Cloning and cellular distribution of a group II phospholipase A2 expressed in the heart. J Mol Cell Cardiol. Aug 1997;29(8):2095-2106.
**18.** Mullins JJ, Peters J, Ganten D. Fulminant hypertension in transgenic rats harbouring the mouse Ren-2 gene. Nature. Apr 5 1990;344(6266):541-544.
**19.** Schroen B, Heymans S, Sharma U, Blankesteijn WM, Pokharel S, Cleutjens JP, Porter JG, Evelo CT, Duisters R, van Leeuwen RE, Janssen BJ, Debets JJ, Smits JF, Daemen MJ, Crijns HJ, Bornstein P, Pinto YM. Thrombospondin-2 is essential for myocardial matrix integrity: increased expression identifies failure-prone cardiac hypertrophy. Circ Res. Sep 3 2004;95(5):515-522.
**20.** Pinto YM, Pinto-Sietsma SJ, Philipp T, Engler S, Kossamehl P, Hocher B, Marquardt H, Sethmann S, Lauster R, Merker HJ, Paul M. Reduction in left ventricular messenger RNA for transforming growth factor beta(1) attenuates left ventricular fibrosis and improves survival without lowering blood pressure in the hypertensive TGR(mRen2)27 Rat. Hypertension. Nov 2000;36(5):747-754.
**21.** Hill JA, Karimi M, Kutschke W, Davisson RL, Zimmerman K, Wang Z, Kerber RE, Weiss RM. Cardiac hypertrophy is not a required compensatory response to short-term pressure overload. Circulation. Jun 20 2000;101(24):2863-2869.
**22.** Heymans S, Schroen B, Vermeersch P, Milting H, Gao F, Kassner A, Gillijns H, Herijgers P, Flameng W, Carmeliet P, Van de Werf F, Pinto YM, Janssens S. Increased cardiac expression of tissue inhibitor of metalloproteinase-1 and tissue inhibitor of metalloproteinase-2 is related to cardiac fibrosis and dysfunction in the chronic pressure-overloaded human heart. Circulation. Aug 23 2005;112(8):1136-1144.
**23.** Guha M, Xu ZG, Tung D, Lanting L, Natarajan R. Specific down-regulation of connective tissue growth factor attenuates progression of nephropathy in mouse models of type 1 and type 2 diabetes. Faseb J. Oct 2007;21(12):3355-3368.
**24.** Sayed D, Hong C, Chen IY, Lypowy J, Abdellatif M. MicroRNAs play an essential role in the development of cardiac hypertrophy. Circ Res. Feb 16 2007;100(3):416-424.
**25.** Wienholds E, Kloosterman WP, Miska E, Alvarez-Saavedra E, Berezikov E, de Bruijn E, Horvitz HR, Kauppinen S, Plasterk RH. MicroRNA expression in zebrafish embryonic development. Science. Jul 8 2005;309(5732):310-311.
**26.** Eulalio A, Behm-Ansmant I, Izaurralde E. P bodies: at the crossroads of post-transcriptional pathways. Nature reviews. Jan 2007;8(1):9-22.
**27.** van Rooij E, Sutherland LB, Qi X, Richardson JA, Hill J, Olson EN. Control of stress-dependent cardiac growth and gene expression by a microRNA. Science. Apr 27 2007;316(5824):575-579.
**28.** Zhao Y, Ransom JF, Li A, Vedantham V, von Drehle M, Muth AN, Tsuchihashi T, McManus MT, Schwartz RJ, Srivastava D. Dysregulation of cardiogenesis, cardiac conduction, and cell cycle in mice lacking miRNA-1-2. Cell. Apr 20 2007;129(2):303-317.
**29.** Boutz PL, Chawla G, Stoilov P, Black DL. MicroRNAs regulate the expression of the alternative splicing factor nPTB during muscle development. Genes Dev. Jan 1 2007;21(1):71-84.
**30.** Kubota S, Kondo S, Eguchi T, Hattori T, Nakanishi T, Pomerantz RJ, Takigawa M. Identification of an RNA element that confers post-transcriptional repression of connective tissue growth factor/hypertrophic chondrocyte specific 24 (ctgf/hcs24) gene: similarities to retroviral RNA-protein interactions. Oncogene. Sep 28 2000;19(41):4773-4786.
**31.** Xiao J, Luo X, Lin H, Zhang Y, Lu Y, Wang N, Zhang Y, Yang B, Wang Z. MicroRNA miR-133 represses HERG K+ channel expression contributing to QT prolongation in diabetic hearts. J Biol Chem. Apr 27 2007; 282 (17) : 12363-12367.

## Claims

1. A method for counteracting, treating, diminishing, delaying and/or preventing fibrosis, preferably cardiac fibrosis, in a subject, the method comprising:
- increasing the expression, amount and/or activity of miRNA-30 and/or miRNA-133, or a functional part or derivative thereof, within said subject, and/or
- increasing interaction of CTGF mRNA with miRNA-30 and/or miRNA-133, or with a functional part or derivative thereof, within said subject.

2. A method according to claim 1, comprising administering pri-miRNA-30 and/or pri-miRNA-133 and/or pre-miRNA-30 and/or pre-miRNA-133 and/or miRNA-30 and/or miRNA-133, or a functional part or derivative thereof, to said subject.

3. Pri-miRNA-30 and/or pri-miRNA-133 and/or pre-miRNA-30 and/or pre-miRNA-133 and/or miRNA-30 and/or miRNA-133, or a functional part or derivative thereof, or a compound capable of increasing or restoring the expression, amount and/or activity of miRNA-30 and/or miRNA-133 in a cell, or a compound capable of increasing interaction of CTGF mRNA with miRNA-30 and/or miRNA-133, for use in counteracting, treating, diminishing, delaying and/or preventing fibrosis, preferably cardiac fibrosis.

4. Use of pri-miRNA-30 and/or pri-miRNA-133 and/or pre-miRNA-30 and/or pre-miRNA-133 and/or miRNA-30 and/or miRNA-133, or a functional part or derivative thereof, or use of a compound capable of increasing or restoring the expression, amount and/or activity of miRNA-30 and/or miRNA-133 in a cell, or use of a compound capable of increasing interaction of CTGF mRNA with miRNA-30 and/or miRNA-133, for the preparation of a medicament or prophylactic agent against fibrosis, preferably cardiac fibrosis.

5. A method for counteracting, treating, diminishing, delaying and/or preventing fibrosis, preferably cardiac fibrosis, in a subject, comprising:
- administering to the subject a therapeutically effective amount of pri-miRNA-30 and/or pri-miRNA-133 and/or pre-miRNA-30 and/or pre-miRNA-133 and/or miRNA-30 and/or miRNA-133, or a functional part or derivative thereof, and/or
- administering to the subject a therapeutically effective amount of a compound capable of increasing or restoring the expression, amount and/or activity of miRNA-30 and/or miRNA-133 in said subject, and/or
- administering to the subject a therapeutically effective amount of a compound capable of increasing interaction of CTGF mRNA with miRNA-30 and/or miRNA-133.

6. A method for determining whether a subject is suffering from fibrosis, or whether a subject has an increased risk of developing fibrosis as compared to a healthy subject, the method comprising:
- obtaining a sample from said subject;
- determining the amount of pri-miRNA-30 and/or pri-miRNA-133 and/or pre-miRNA-30 and/or pre-miRNA-133 and/or miRNA-30 and/or miRNA-133 in said sample;
- comparing said amount with a reference; and
- determining from said comparison whether or not the subject is suffering from fibrosis or is having an increased risk of developing fibrosis as compared to a healthy subject.

7. A method according to claim 6, wherein said fibrosis comprises cardiac fibrosis.

8. A kit of parts comprising:
- at least one compound capable of specifically binding pri-miRNA-30 and/or pri-miRNA-133 and/or pre-miRNA-30 and/or pre-miRNA-133 and/or miRNA-30 and/or miRNA-133; and
- instructions for use of said compound for determining whether a subject is suffering from fibrosis, preferably cardiac fibrosis, or whether a subject has an increased risk of developing fibrosis, preferably cardiac fibrosis.

9. A kit of parts comprising:
- pri-miRNA-30 and/or pri-miRNA-133 and/or pre-miRNA-30 and/or pre-miRNA-133 and/or miRNA-30 and/or miRNA-133, or a functional part or derivative thereof, and/or
- a compound capable of increasing or restoring the expression, amount and/or activity of miRNA-30 and/or miRNA-133 in a cell, and/or
- a compound capable of increasing interaction of CTGF mRNA with miRNA-30 and/or miRNA-133, and
- instructions for use of said compound(s) for counteracting, treating, diminishing, delaying and/or preventing fibrosis, preferably cardiac fibrosis, in a subject in need thereof.

10. Use of a vector comprising:
- pri-miRNA-30 and/or pri-miRNA-133 and/or pre-miRNA-30 and/or pre-miRNA-133 and/or miRNA-30 and/or miRNA-133, or a functional part or derivative thereof, and/or
- a nucleic acid sequence encoding a compound capable of increasing or restoring expression, amount and/or activity of miRNA-30 and/or miRNA-133 in a cell, and/or
- a nucleic acid sequence encoding a compound capable of increasing interaction of CTGF mRNA with miRNA-30 and/or miRNA-133,
for the preparation of a medicament and/or prophylactic agent for counteracting, treating, diminishing, delaying and/or preventing fibrosis, preferably cardiac fibrosis.

11. Use of an isolated cell capable of overexpressing pri-miRNA-30 and/or pri-miRNA-133 and/or pre-miRNA-30 and/or pre-miRNA-133 and/or miRNA-30 and/or miRNA-133, or a functional part or derivative thereof, for the preparation of a medicament and/or prophylactic agent for counteracting, treating, diminishing, delaying and/or preventing fibrosis, preferably cardiac fibrosis.

12. A pharmaceutical composition comprising:
- pri-miRNA-30 and/or pri-miRNA-133 and/or pre-miRNA-30 and/or pre-miRNA-133 and/or miRNA-30 and/or miRNA-133, or a functional part or derivative thereof, and/or
- a compound capable of increasing or restoring the expression, amount and/or activity of miRNA-30 and/or miRNA-133 in a cell, and/or
- a compound capable of increasing interaction of CTGF mRNA with miRNA-30 and/or miRNA-133,
together with a pharmaceutically acceptable carrier, diluent or excipient.

13. A non-human animal comprising:
- an exogenous nucleic acid sequence comprising pri-miRNA-30 and/or pri-miRNA-133 and/or pre-miRNA-30 and/or pre-miRNA-133 and/or miRNA-30 and/or miRNA-133, or a functional part or derivative thereof, and/or
- an exogenous nucleic acid sequence encoding a compound capable of increasing or restoring the expression, amount and/or activity of miRNA-30 and/or miRNA-133 in a cell, and/or
- an exogenous nucleic acid sequence encoding a compound capable of increasing interaction of CTGF mRNA with miRNA-30 and/or miRNA-133.

14. A method for determining whether a candidate compound is capable of counteracting, treating, diminishing, delaying and/or preventing fibrosis, preferably cardiac fibrosis, the method comprising:
- contacting said candidate compound with a cell, preferably with a cardiac cell, and
- determining whether said candidate compound is capable of increasing the expression, amount and/or activity of miRNA-30 and/or miRNA-133 in said cell.

15. A method for determining whether a candidate compound is capable of counteracting, treating, diminishing, delaying and/or preventing fibrosis, preferably cardiac fibrosis, the method comprising:
- contacting said candidate compound with a cell, preferably with a cardiac cell, and
- determining whether said candidate compound is capable of increasing interaction of CTGF mRNA with miRNA-30 and/or miRNA-133, or with a functional part or derivative thereof, within said cell.
